# FASCICULE DE BREVET EUROPEEN

(11) **EP 0 345 159 B1**
(45) Date de publication et mention de la délivrance du brevet: **10.02.1993**
(21) Numéro de dépôt: 89401503.1
(22) Date de dépôt: 01.06.1989
(51) Int. Cl.: A61M 5/315

(54) **Seringue à injection unique**
Spritze zur einmaligen Injektion
Single injection syringe

(30) Priorité: 03.06.1988 FR 8807412; 30.12.1988 FR 8817474
(43) Date de publication de la demande: 06.12.1989
(73) Titulaire: Clotteau, Jean-Edouard, F-75017 Paris (FR); Pozzi, Didier, F-92190 Meudon (FR); Pozzi, Jean-Pierre, F-92190 Meudon (FR)
(72) Inventeur: Pozzi, Didier, F-92190 Meudon (FR); Pozzi, Jean-Pierre, F-92190 Meudon (FR)
(74) Mandataire: Lhuillier, René

(56) Documents cités:
- EP-A- 0 229 017
- FR-A- 2 205 343
- FR-A- 2 298 340
- FR-A- 2 606 643
- US-A- 4 233 975

## Description

L'invention concerne une seringue à injection unique, c'est-à-dire ne pouvant être utilisée qu'une seule fois, et porte plus précisément sur un dispositif rendant inopérant le joint d'étanchéité après une première injection.

Dans le domaine thérapeutique, l'usage de seringues pour l'injection de liquides dans les tissus ou cavités naturelles du corps est extrêmement répandu. On sait qu'une seringue se compose essentiellement d'un corps cylindrique dans lequel coulisse un piston, corps dont la base porte un ajustage de forme appropriée auquel peut être adaptée une aiguille creuse, par exemple en acier ou en nickel, ledit corps formant donc un réservoir pour le liquide à injecter. Le piston peut avoir une structure ou une composition différente selon les modèles. Il est en tout cas muni à une de ses extrémités d'un joint qui assure l'étanchéité avec le corps de la seringue, et à son autre extrémité qui est toujours extérieure au corps de seringue, d'une tête de piston pour faciliter sa manipulation. Cette seringue classique permet d'assurer de façon habituelle les opérations nécessaires à une injection courante, c'est-à-dire en premier lieu le dégommage du joint au fond de la seringue puis en exerçant une traction sur le piston pour l'extraire du corps de la seringue, l'aspiration d'une certaine quantité de liquide. Ensuite la seringue étant en position renversée, c'est-à-dire aiguille vers le haut, une petite pression sur le piston provoque le refoulement de l'air qui pourrait subsister dans le réservoir, cette opération étant éventuellement suivie d'une réaspiration complémentaire de liquide et à nouveau du refoulement indispensable de l'air.

Avant l'injection proprement dite, il est prévu enfin une légère réaspiration après piquage pour contrôle.

On connaît des seringues à injection unique utilisées pour les vaccinations, qui sont préremplies en laboratoire, et dont on ne peut plus réaspirer à nouveau de liquide après l'injection (voir par example le document EP-A-0229017 qui est défini dans le préambule de la revendication 1). Mais comme la plupart des seringues utilisées ne sont pas préremplies,et puisque la manoeuvre aller et retour du piston doit pouvoir s'effectuer pour les opérations décrites plus haut ou pour une manoeuvre à vide il n'est pas possible d'adopter ce système. Un objet de la présente invention consiste donc en un dispositif adaptable à toute seringue, qui permet d'assurer toutes les opérations nécessaires à une injection complète et incluant par conséquent les allers et retours du piston ou ses manoeuvres à vide évoquées précédemment, mais qui interdit rigoureusement un réemploi pour une seconde injection, ledit dispositif consistant à rendre inopérant le joint d'étanchéité à l'issue de la première injection en mettant à profit l'action de la pression qu'exerce le liquide, durant l'injection, sur une partie souple du joint, pour désolidariser ledit joint de son piston d'entraînement, ou pour annihiler sa fonction d'organe d'étanchéité.

L'invention concerne par conséquent une seringue à injection unique comportant un corps de seringue cylindrique à la base duquel peuvent être fixées différentes sortes d'aiguilles de prélèvement et/ou d'injection, ainsi qu'un piston coulissant à l'intérieur du corps de seringue dont l'extrémité porte un joint glissant de façon étanche sur la paroi intérieure de la seringue, seringue selon laquelle le joint comporte au moins un organe susceptible d'être déformé ou déplacé à partir de sa position d'équilibre sous l'action de la pression qu'exerce le liquide contenu dans la seringue lors de la phase d'injection le piston coulissant étant associé audit joint par l'intermédiaire de pièces qui réagissent à la déformation ou au déplacement dudit organe et qui rendent le joint inopérant pour un nouveau remplissage à l'issue de l'injection.

Selon des caractéristiques particulières de l'invention, le joint est une pièce cylindrique creuse dont la face inférieure est formée d'une membrane souple qui constitue l'organe déformable et qui assure l'étanchéité entre le corps de seringue et la chambre à l'intérieur du joint et dont la partie supérieure de forme annulaire prend appui contre le piston, lequel piston se prolonge, au travers de l'orifice de la partie annulaire du joint, par une tige axiale de diamètre inférieur à celui de l'orifice et se terminant par une tête formant un épaulement avec la tige.

Selon une caractéristique particulière de l'invention,la membrane souple ou l'organe déplaçable porte un orifice normalement fermé de façon étanche par une pièce obturatrice qui suit le mouvement de déformation ou de déplacement de ladite membrane ou dudit organe sous l'effet de la pression développée lors de la phase d'injection, mais qui est retenue par des moyens de blocage dans une position où elle ouvre l'orifice, rompant l'étanchéité entre le dessus et le dessous du joint, dès que la membrane ou l'organe revient dans sa position initiale.,

D'autres caractéristiques particulières et les avantages de l'invention apparaîtront à la lecture de la description qui va suivre d'exemples de réalisation qui font référence aux dessins annexés qui représentent :
figure 1, une vue schématique en élévation d'une seringue équipée, en position repos ;
figure 2, la même seringue pendant une phase d'injection ;
figures 3 et 4, une autre forme de réalisation également dans les deux positions ;
figures 5 et 6, une autre variante de seringue équipée d'un dispositif de désolidarisation du joint en position repos et en position injection ;
figures 7 et 8, une vue schématique en élévation d'une seringue équipée d'un dispositif de perforation du joint, en position repos et en position injection ;
figure 9, une autre variante de réalisation.

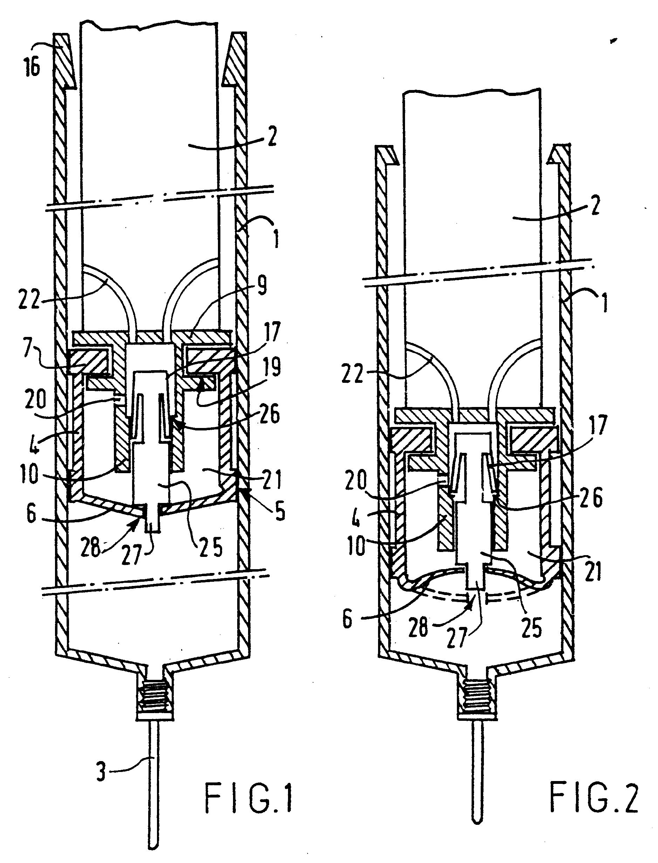

La seringue représentée à la figure 1 se compose essentiellement d'un corps de seringue cylindrique 1 à la base duquel est fixée une aiguille 3 de prélèvement ou d'injection, d'un piston 2 coulissant à l'intérieur du corps de seringue et d'un joint d'étanchéité 4 monté en bout du piston et glissant de façon étanche sur la paroi intérieure de la seringue. Le piston ne peut être sorti du corps de la seringue 1 à cause d'un dispositif antiretour 16 évitant toute intervention sur le dispositif. Le joint 4 se présente comme une pièce cylindrique creuse dont la paroi extérieure est pourvue de nervures 5 moulées dans la masse, formant étanchéité sur la paroi interne du corps de seringue 1. La face inférieure du joint, en regard de l'orifice du corps de seringue forme une membrane souple 6 d'épaisseur réduite par rapport au reste, membrane qui, en position d'équilibre, est sensiblement horizontale et assure l'étanchéité entre le corps de seringue et une chambre 21 intérieure au joint. Du côté opposé, la partie supérieure 7 du joint de forme annulaire, en appui contre un épaulement 9 du piston, présente une épaisseur plus importante et est pourvue d'un orifice central 8. La face inférieure de cette partie supérieure 7 du joint est en appui sur un épaulement 19 venant d'une tige cylindrique creuse 10 qui prolonge le piston 2 et se développe à l'intérieur du joint. Le joint 4 est ainsi rendu solidaire du piston 2. Dans l'intérieur de la tige creuse 10 est placée une pièce obturatrice 25, de forme générale allongée, et dont la section est cylindrique ou en croisillon. Cette pièce est susceptible de se déplacer avec un faible jeu à l'intérieur du puits qui constitue la tige 10 qui porte par ailleurs un épaulement interne 26 délimitant dans ladite tige un puits inférieur de plus faible section que le puits supérieur au niveau de la partie annulaire 7 du joint. L'épaulement pourrait être aussi un bossage intéressant ou non toute la circonférence du puits. La pièce obturatrice 25 se termine vers le bas par une tête d'obturation 27 qui ferme un petit orifice 28 prévu dans la membrane 6. A sa partie supérieure, la pièce obturatrice 25 porte des lamelles élastiques 17 maintenues repliées quand elles sont appliquées contre la paroi intérieure de la tige 10, et qui jouent le rôle d'organe antiretour, comme on le verra plus loin. On notera que la tige 10 est traversée par des lumières 20 faisant communiquer la chambre intérieure 21 du joint 4 avec le puits intérieur de la tige et de là par des lumières 22 prévues dans le piston, avec la seringue, au-dessus de l'épaulement du piston. La position représentée sur cette figure 1 correspond à la phase préliminaire d'injection. La tête 27 de la pièce obturatrice 25 ferme de façon étanche la membrane 6, en suivant son mouvement.

Dès que l'on passe en phase d'injection, comme représenté à la figure 2, sous l'effet de la pression développée, la membrane 6 va se déformer de la position représentée en pointillés à la position représentée en traits pleins. La pièce obturatrice 25 suit ce mouvement et les lamelles élastiques 17 vont alors échapper au puits inférieur et se déployer dans le puits supérieur. Elles seront alors bloquées par l'épaulement 26 rendant impossible tout retour de la pièce vers sa position initiale. Cette fonction antiretour ne prend effet que si la pression dans le réservoir a atteint une valeur maximale déterminée, le déplacement de la pièce obturatrice restant réversible pour toutes les valeurs inférieures de la pression. A l'issue de l'injection, le joint 4 reste solidaire du piston. Mais la partie déformée de la membrane 6 n'étant plus soumise à la surpression, a repris sa forme initiale. Cependant la tête d'obturation 27 reste écartée de la membrane et l'orifice 28 est dégagé. La chambre 21 étant reliée à l'atmosphère par les lumières 22 pratiquées dans le piston 2, et par les lumières 20, communique aussi avec le bas de la seringue par l'orifice 28. L'étanchéité est donc rompue entre le dessus et le dessous du joint. Ainsi le remplissage du dispositif d'injection par aspiration de liquide ou par action d'un liquide en surpression, de même que l'injection, sont rendus impossibles.

Selon une variante non représentée, on peut considérer que le fond 6 reste rigide, mais que ce sont les parois latérales du joint 4, au-dessous de sa partie annulaire 7, qui se déforment et se replient lors de la phase d'injection et déplacent la base du joint et la pièce obturatrice vers le haut.

Selon encore une autre variante non représentée la pièce obturatrice n'est pas munie de lamelles élastiques antiretour 17 mais se colle en position haute contre le piston grâce à un adhésif ou une colle appropriée localisés sur le piston et/ou ladite pièce.

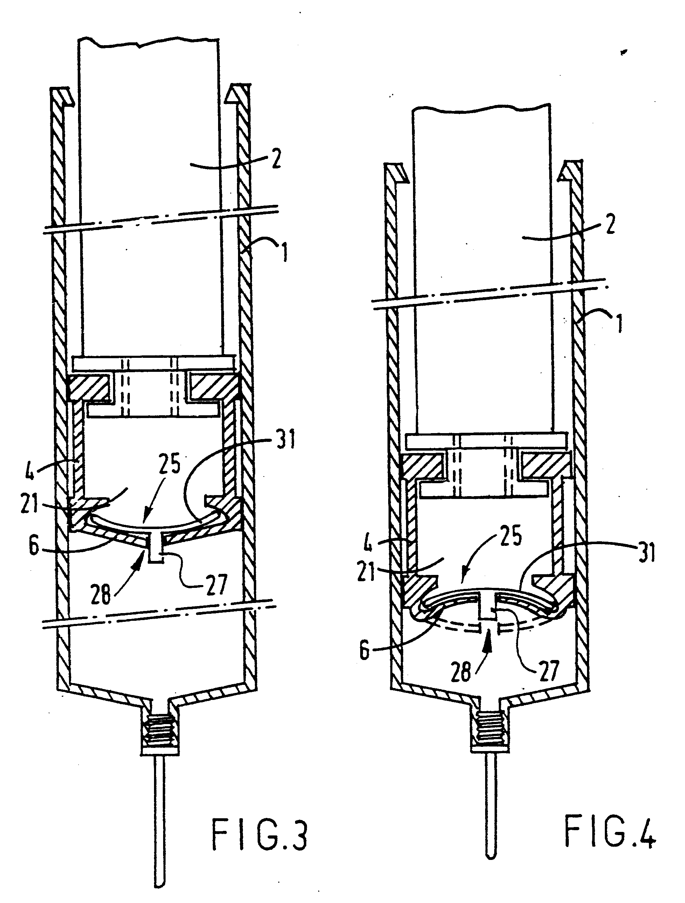

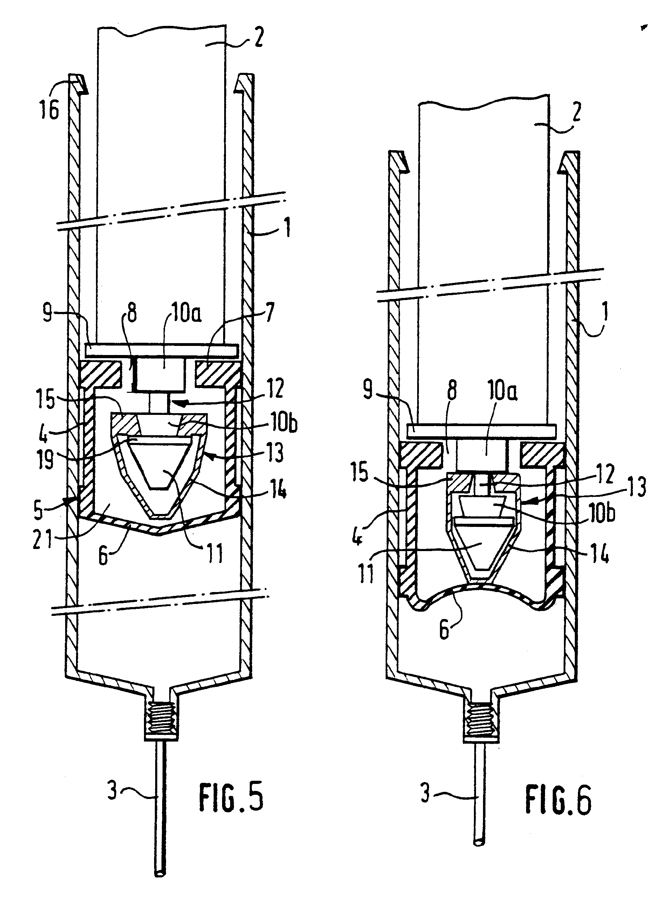

On a représenté aux figures 3 et 4 encore une autre forme de réalisation, toujours dans les deux phases habituelles de fonctionnement. Cette fois la pièce obturatrice 25 est constituée d'une lame cloquante 31 dont la concavité est dirigée vers le bas, dans le même sens que celle de la membrane 6 non déformée du joint 4 à l'intérieur duquel elle est maintenue. La lame cloquante 31 porte en son centre une tête d'obturation 27 qui coopère aussi avec l'orifice 28. En phase initiale (figure 3) la lame 31 est en position cambrée, comme représentée, pour laquelle l'orifice 28 est obturé. Lors de la phase d'injection illustrée à la figure 6, la surpression dans la seringue est suffisante pour que la déformation de la membrane 6 pousse la lame cloquante 31 vers le haut. Sa concavité change donc de sens et subsiste dans cette position. Lorsque la membrane reprend sa position initiale, à la fin de l'injection, l'orifice 28 n'est plus fermé par la tête d'obturation 27 puisque la lame est restée cambrée en position haute. On notera que le frottement entre la tête 27 et les bords de la membrane autour de son orifice 28 est insuffisant pour ramener la lame cloquante 31 de la position de la figure 6 à celle de la figure 5, sous la seule traction de la membrane 6 revenant à sa position initiale.

Selon une variante non représentée, la lame cloquante, au lieu d'être associée au joint 4 pourrait être associée à un organe rigide déplaçable.

Pour la variante des figures 3 et 4 le fonctionnement et le résultat obtenus sont les mêmes que pour le cas décrit en référence aux figures 1 et 2.

On a décrit l'invention en se référant à un joint 4 qui se présente comme une pièce cylindrique creuse dont la paroi extérieure est pourvue de nervures moulées dans la masse formant étanchéité sur la paroi interne du corps de seringue 1, et dont la partie inférieure est une membrane déformable 6. On peut considérer que l'invention s'applique également à un piston de seringue muni d'au moins un joint rapporté ou profilé pour faire joint lui-même, qui serait équipé d'un organe déformable jouant le même rôle que celui décrit plus haut ou d'une pièce déplaçable à la place du déplacement possible du fond du joint, évoqué précédemment, sous l'effet de la pression.

Pour améliorer encore la sécurité de non réutilisation, il peut être avantageux de prévoir une zone de faiblesse, par exemple au niveau de la tête d'obturation 27. Une tentative de remise en position initiale de la pièce obturatrice 25 par traction entraînerait la rupture entre ladite tête et la pièce.

On va décrire maintenant, en référence aux figures 5 et 6,une variante de réalisation dans laquelle cette fois la membrane 6 du joint 4 n'est plus perforée et reste étanche mais renferme un dispositif qui désolidarise le joint du piston.

On voit à la figure 5, dans laquelle les mêmes références sont utilisées pour les mêmes pièces que celles décrites pour la figure 1, que la tige axiale 10 qui prolonge le piston présente une partie 10a de diamètre sensiblement inférieur au diamètre de l'orifice 8, au niveau dudit orifice, et que cette tige se termine par une tête conique 11 de plus grand diamètre formant un épaulement 19 avec la tige, tête conique dont la pointe est orientée vers la membrane 6 du joint, l'épaulement restant inférieur au diamètre de l'orifice 8. La tige 10 porte une gorge 12, entre l'épaulement 9 et la tête conique 11, laquelle tête étant conique pour permettre un montage plus aisé du piston sur l'agrafe. La portion 10b de la tige entre cette gorge et la pièce conique a été chanfreinée pour présenter un profil légèrement conique comme on le voit à la figure 5. Une agrafe élastique 13 formée de bras 14 qui font retour par des mâchoires 15 vers le centre de la seringue est disposée à l'intérieur du joint 4 et coiffe la tête conique 11. Elle est représentée déployée à la figure 5 et ses mâchoires 15 dont les extrémités ont un profil conique correspondant à celui de la partie 10b, sont en appui contre la partie 10b de la tige 10 et contre l'épaulement supérieur de la pièce conique.

Si on exerce sur le piston 2 une pression vers le bas, l'épaulement 9 pousse le joint par sa face supérieure 7. Si au contraire, on exerce sur le piston une traction vers le haut, à partir de la position représentée sur la figure 5, par exemple pour assurer l'aspiration de liquide dans la seringue, les mâchoires 15 de l'agrafe, maintenues par la partie 10b selon un écartement supérieur au diamètre de l'orifice 8, viendront en butée contre la partie annulaire 7 du joint et permettront d'entraîner ce dernier avec le piston. La façon dont sont ainsi solidarisés le piston et le joint autorise les manoeuvres préalables à l'injection évoquées précédemment.

La figure 6 illustre la phase finale d'injection de liquide alors que le piston 2 descend vers le fond de la seringue 1. A ce moment là la pression du liquide dans la seringue sous l'effet de la poussée du joint 4 provoque la déformation de la membrane 6 qui se gonfle vers l'intérieur du joint 4. La membrane appuie alors sur l'agrafe 13 en la déplaçant vers le haut jusqu'à ce que ses mâchoires 15 échappent à la portion 10b de la tige, et, du fait de l'élasticité des bras 14, viennent se resserrer dans la gorge 12. L'empattement de l'agrafe est alors réduit à une dimension inférieure à celle de l'orifice 8 et on comprend alors qu'à l'issue de l'injection, si on veut retirer le piston de la seringue, l'agrafe passera par l'orifice 8 et le joint restera au fond de la seringue. On notera que même si cette désolidarisation du joint par retrait de l'agrafe est intervenue en début de la course d'enfoncement du piston, l'injection n'en sera pas perturbée et pourra se poursuivre normalement grâce à la poussée de l'épaulement 9 sur le joint. En fin de course la tête conique 11 du piston 2 par son action sur la membrane 6, appliquera cette dernière sur le fond de la seringue, permettant l'injection de tout le liquide. La déformation que prendra le joint dans cette phase finale est sans importance,puisque de toute façon l'agrafe ne pourra plus coopérer avec la partie annulaire 7 du joint qui restera au fond.

Au cas où pour une raison quelconque, les mâchoires de l'agrafe 13 n'échapperaient pas à la portion 10b de la tige, sous l'action de la pression, la désolidarisation interviendrait néanmoins de toute façon quand l'agrafe viendrait en butée au travers de la membrane sur le fond de la seringue, en fin de course du mouvement du piston.

L'agrafe 13 visible aux figures 5 et 6 peut avoir un profil différent de celui représenté et notamment une face inférieure de laquelle partent les bras 14, sensiblement plus importante, pour éviter de glisser sur la membrane 6 et coopérer plus facilement avec elle à la fin de l'injection.

On décrit maintenant encore une autre variante de réalisation dans laquelle la membrane 6 est étanche comme dans les cas ci-dessus et non munie d'un orifice, mais où par contre elle est perforable.

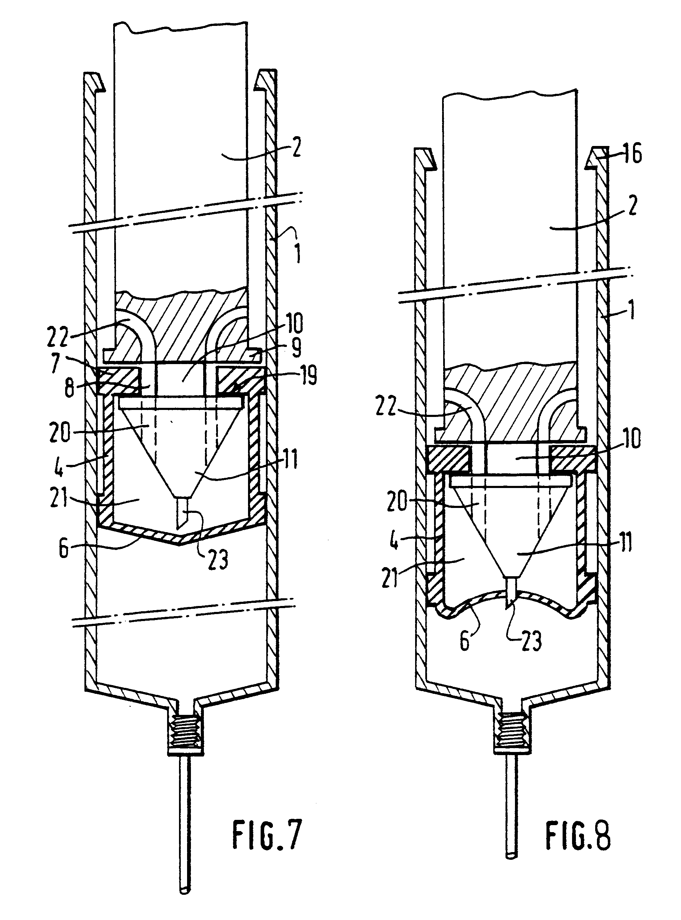

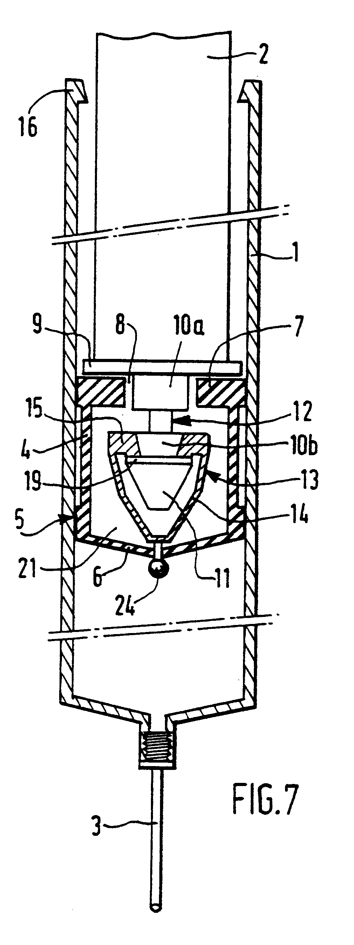

Cette variante est décrite en référence aux figures 7 et 8, qui montre que la tête conique 11 toujours solidaire du piston 2 par la tige 10, et logée à l'intérieur du joint 4, présente une dimension plus importante. Son épaulement 19 par rapport à la tige vient en butée contre la partie inférieure annulaire 7 du joint 4, en coiffant l'orifice 8. Cette partie annulaire 7 du joint est ainsi pincée entre l'épaulement 9 et l'épaulement 19 ; le joint et le piston sont ainsi rendus inséparables. Comme dans les cas de figure 1 à 4, la tête conique 11 est traversée par des lumières 20 faisant communiquer la chambre intérieure 21 du joint 4 avec l'orifice 8. D'autres lumières 22 sont prévues dans le piston lui-même pour qu'il y ait libre communication d'air entre la chambre 21 et la seringue, au-dessus de l'épaulement 9 du piston. En outre la tête conique 11 est pourvue à son extrémité d'une pointe 23 orientée en direction de la membrane 6, pointe qui pourrait être aussi remplacée par une pièce tranchante de forme quelconque.

Dans la position représentée à la figure 7 qui correspond à une phase opératoire préliminaire à l'injection proprement dite, la membrane 6 occupe une position d'équilibre qui la tient à l'écart de la pointe 23. Dès qu'on est en phase d'injection, et que la pression du liquide devient suffisante pour déformer la membrane 6, cette dernière vient s'appliquer fortement contre la pointe 23 et se trouve perforée (figure 8). Néanmoins on peut poursuivre normalement l'injection puisque la pointe osbtrue la perforation et empêche le liquide de pénétrer dans la chambre 21. A l'isue de l'injection, le joint 4 reste solidaire du piston, comme on l'a vu plus haut, mais si l'on veut effectuer une nouvelle aspiration de liquide en vue d'une autre injection, la perforation de la membrane 6 rendra l'opération impossible. En effet l'air aura pu passer par les lumières 20, 22 et ladite perforation, et pénétrer dans le bas de la seringue, empêchant définitivement d'appliquer toute dépression et de pouvoir aspirer du liquide. Le dispositif anti-retour 16 ou tout autre système obstruant la seringue en partie haute mais laissant coulisser le piston empêche en outre toute intervention sur la seringue dans touts les cas décrits.

Selon encore une autre variante de réalisation illustrée à la figure 9, l'agrafe 13, utilisée selon le premier mode de réalisation conformément aux figures 5 et 6 possède à sa partie inférieure une excroissance 24 qui peut avoir la forme d'une pointe à bout arrondi. Au montage du joint, on fait en sorte que cette excroissance 24 se trouve de l'autre côté de la membrane 6 par rapport à l'agrafe, la pointe de l'excroissance traversant un trou central prévu sur la membrane.

L'agrafe et la membrane sont ainsi solidarisées.

Le fonctionnement est identique à celui décrit concernant la réalisation des figures 5 et 6, c'est-à-dire que le gonflement de la membrane 6 lors de l'injection repoussera l'agrafe 13 vers le haut, dont les mâchoires pourraient se resserrer dans la gorge 12. Si un utilisateur cherche à extraire le piston 2, l'entraînement de l'agrafe 13 fera sortir l'excroissance 24 en débouchant le trou prévu sur la membrane, et il subsistera ce trou dans le joint 4 qui constituera, en plus du fait que le joint et le piston sont détachés l'un de l'autre, une détérioration complémentaire du joint le rendant inopérant. On notera que le retrait de l'excroissance au travers de la membrane est possible du fait de forces de frottement du joint dans le corps de seringue supérieures à la force de retenue de l'excroissance par le trou de la membrane. On rejoint ainsi les cas illustrés aux figures 1 à 4.

Un avantage complémentaire de cette variante réside en ce qu'ainsi, l'agrafe se trouve au montage, parfaitement positionnée par rapport au joint.

Les dispositifs ainsi décrits sont adaptables à toutes sortes de seringues. Hormis le fait qu'ils sont parfaitement efficaces et rendent les seringues ainsi équipées, rigoureusement non réutilisables, ils sont simples à réaliser et par conséquent peu coûteux.

## Revendications

1. Seringue à injection unique comportant un corps de seringue cylindrique à la base duquel peut être fixée une aiguille de prélèvement et/ou d'injection, et un piston coulissant à l'intérieur du corps de seringue dont l'extrémité porte un joint glissant de façon étanche sur la paroi intérieure de la seringue, caractérisée en ce que le joint (4) comporte au moins un organe (6) susceptible d'être déformé ou déplacé à partir de sa position d'équilibre sous l'action de la pression qu'exerce le liquide contenu dans la seringue lors de la phase d'injection et en ce que le piston coulissant (2) est associé audit joint par l'intermédiaire de pièces (11, 13, 23, 27) qui réagissent à la déformation ou au déplacement dudit organe et qui rendent le joint inopérant pour un nouveau remplissage à l'issue de l'injection.

2. Seringue selon la revendication 1,
caractérisée en ce que le joint (4) est une pièce cylindrique creuse dont la face inférieure est formée d'une membrane souple (6) qui constitue l'organe déformable et qui assure l'étanchéité entre le corps de seringue (1) et la chambre (21) à l'intérieur du joint et dont la partie supérieure (7) de forme annulaire prend appui contre le piston (2).

3. Seringue selon les revendications 1 et 2,
caractérisée en ce que la membrane souple (6) ou l'organe déplaçable portent un orifice (28) normalement fermé de façon étanche par une pièce obturatrice (25, 26) qui suit le mouvement de déformation ou de déplacement de ladite membrane ou dudit organe, sous l'effet de la pression développée lors de la phase d'injection, mais qui est retenue par des moyens de blocage (17, 31) dans une position où elle ouvre l'orifice, rompant l'étanchéité entre le dessus et le dessous de joint, dès que la membrane ou l'organe revient dans sa position initiale.

4. Seringue selon la revendication 3,
caractérisée en ce que la pièce obturatrice (25) est montée à l'intérieur d'une tige creuse (10) portant un épaulement interne (26), qui prolonge le piston (2), et se termine par une tête d'obturation (27) qui ferme un orifice (28) prévu dans la membrane (6).

5. Seringue selon les revendications 3 et 4,
caractérisée en ce que les moyens de blocage de la pièce obturatrice (25) sont constitués de lamelles élastiques portées par ladite pièce, et repliées à l'intérieur de la tige creuse (10), lesdites lamelles venant s'accrocher sur l'épaulement (26) quand la pièce obturatrice a été déplacée par la déformation de la membrane (6).

6. Seringue selon les revendications 3 et 4,
caractérisée en ce que les moyens de blocage de la pièce obturatrice (25) sont constitués de colle ou adhésifs localisés sur le piston et/ou ladite pièce.

7. Seringue selon les revendications 1 et 3,
caractérisée en ce que la pièce obturatrice (25) est constituée d'une lame cloquante (31) maintenue à l'intérieur du joint (4), dont la concavité est dirigée dans le même sens que celle de la membrane (6) non déformée du joint, et qui porte en son centre une tête d'obturation (27) qui ferme un orifice (28) prévu dans la membrane.

8. Seringue selon les revendications 1, 2 et 3
caractérisée en ce que les parois latérales du joint (4) au-dessous de sa partie annulaire (7) sont déformables, tandis que le fond (6) reste rigide.

9. Seringue selon la revendication 3,
caractérisée en ce qu'on prévoit, sur la pièce obturatrice (25) au niveau de la tête d'obturation (27), une zone de faiblesse susceptible d'entraîner sa rupture, en cas de tentative de réutilisation.

10. Seringue selon les revendications 1 et 2,
caractérisée en ce que le piston (2) se prolonge au travers de l'orifice (8) de la partie annulaire (7) du joint, par une tige axiale (10) de diamètre inférieur à celui de l'orifice et se terminant par une tête (11) formant un épaulement (19) avec la tige.

11. Seringue selon les revendications 1 et 10,
caractérisée en ce qu'est montée au-dessus de la tête (11), à l'intérieur du joint (4), une agrafe élastique (13) formée de bras (14) qui font retour vers la tige axiale (10) par des mâchoires (15) en prenant appui contre une partie inférieure (10b) de cette tige, une gorge (12) étant prévue sur la tige sensiblement à égale distance entre le piston (2) et la tête.

12. Seringue selon la revendication 11,
caractérisée en ce que la partie (10b) de la tige (10) présente un profil légèrement conique correspondant au profil des extrémités des mâchoires (15).

13. Seringue selon les revendications 1, 2 et 11, caractérisée en ce que l'agrafe (13) reste en position déployée pour laquelle ses mâchoires (15) présentent un écartement supérieur au diamètre de l'orifice (8), tant que la membrane souple (6) n'a pas subi de déformation.

14. Seringue selon les revendications 1, 2 et 11, caractérisée en ce que la déformation de la membrane souple (6) assure le déplacement de l'agrafe (13), le repli des mâchoires (15) dans la gorge (12), et la désolidarisation du joint (4) et du piston (2).

15. Seringue selon les revendications 1, 2 et 11, caractérisée en ce qu'en fin de course du mouvement du piston l'agrafe (13) vient en butée au travers de la membrane sur le bord de la seringue, provoquant le repli des mâchoires (15) dans la gorge (12) et la désolidarisation du joint (4) et du piston (2).

16. Seringue selon la revendication 10,
caractérisée en ce que l'épaulement (19) de la tête (11) a un diamètre supérieur à celui de l'orifice (8), la tête étant traversée de lumières (20) faisant communiquer la chambre intérieure (21) du joint (4) avec l'orifice (8), et en ce que d'autres lumières (22) sont prévues dans le piston (2).

17. Seringue selon les revendications 1 et 16,
caractérisée en ce que la tête (11) est pourvue à son extrémité inférieure d'une pièce tranchante (23) orientée en direction de la membrane et effacée à l'intérieur de la chambre (21) tant que la membrane souple (6) n'a pas subi de déformation.

18. Seringue selon les revendications 1 et 17,
caractérisée en ce que la déformation de la membrane souple (6) entraîne sa perforation sur la pièce tranchante (23) et annihile ainsi la fonction d'étanchéité du joint (4).

19. Seringue selon les revendications 1, 2 et 11 caractérisée en ce que l'agrafe (13) possède à sa partie inférieure une excroissance (24) traversant un trou prévu dans la membrane (6) du joint (4) assurant l'étanchéité.

## Patentansprüche

1. Spritze zur einmaligen Injektion mit einem zylindrischen Spritzenkörper, an dessen Basis eine Nadel für die Entnahme und/oder Injektion befestigt werden kann, und mit einem im Inneren dieses Spritzenkörpers verschiebbaren Kolben, dessen Endbereich eine Dichtung trägt, die abdichtend an der Innenwand der Spritze gleitet, dadurch gekennzeichnet, daß die Dichtung (4) zumindest ein Teil (4) aufweist, das ausgehend von seiner Gleichgewichtsposition unter der Wirkung des Drucks, den die in der Spritze befindliche Flüssigkeit während des Vorgangs der Injektion ausübt, deformierbar oder verschiebbar ist, und daß der verschiebbare Kolben (2) mit der Dichtung über Teilstücke (11, 13, 23, 27) zusammenhängt, die auf die Deformation oder Verschiebung des genannten Teils wirken und die die Dichtung unwirksam werden lassen für ein neues Füllen nach einer Injektion.

2. Spritze nach Anspruch 1, dadurch gekennzeichnet, daß die Dichtung (4) ein zylindrisches Hohlteil ist, dessen Unterseite durch eine weiche Membran (6) gebildet wird, die das deformierbare Teil bildet, eine Abdichtung zwischen dem Spritzenkörper (1) und der Kammer (21) innerhalb der Dichtung sicherstellt und deren oberer Teilbereich (7) mit Ringform am Kolben (2) anliegt.

3. Spritze nach den Ansprüchen 1 und 2, dadurch gekennzeichnet, daß die weiche Membran (6) oder das verschiebbare Teil eine Öffnung (28) haben, die normalerweise abgedichtet durch ein Schließteil (25, 26) geschlossen ist, das der Deformations- oder Verschiebungsbewegung der genannten Membran oder des genannten Teils folgt unter der Wirkung des sich während der Injektion einstellenden Drucks, das aber durch Blockiermittel (17, 31) in einer Position zurückgehalten wird, in der es die Öffnung freigibt und die Abdichtung zwischen den Bereichen oberhalb und unterhalb der Dichtung aufhebt, während die Membran oder das Teil in seine Ausgangsposition zurückgeht.

4. Spritze nach Anspruch 3, dadurch gekennzeichnet, daß das Schließteil (25) im Inneren eines Stutzens (10) angeordnet ist, der eine Innenschulter (26) aufweist und den Kolben verlängert, und daß das Schließteil (25) an seinem Ende einen Verschlußkopf (27) hat, der die in der Membran (6) vorgesehene Öffnung (28) schließt.

5. Spritze nach den Ansprüchen 3 und 4, dadurch gekennzeichnet, daß die Blockiermittel des Schließteils (25) elastische Lamellen sind, die von dem Schließteil getragen werden und in den Innenraum des Stutzens (10) gefaltet sind, und daß diese Lamellen hinter die Innenschulter (26) greifen, wenn das Schließteil durch die Deformation der Membran (6) verschoben wurde.

6. Spritze nach den Ansprüchen 3 und 4, dadurch gekennzeichnet, daß die Blockiermittel des Schließteils (25) durch einen Klebstoff oder ein Adhäsionsmittel gebildet sind, das am Kolben und/oder am genannten Schließteil angeordnet ist.

7. Spritze nach den Ansprüchen 1 und 3, dadurch gekennzeichnet, daß das Schließteil (25) eine Schnappfeder (31) aufweist, die im Innenraum der Dichtung (4) gehalten ist und deren Krümmung im selben Sinne gerichtet ist wie diejenige der nicht deformierten Membran (6) der Dichtung, und daß diese Schnappfeder in ihrem Mittelbereich einen Verschlußkopf (27) trägt, der eine in der Membran vorgesehene Öffnung (28) verschließt.

8. Spritze nach den Ansprüchen 1, 2 und 3, dadurch gekennzeichnet, daß die seitlichen Wände der Dichtung (4) unterhalb ihres Ringbereichs deformierbar sind, wohingegen der Boden (6) steif bleibt.

9. Spritze nach Anspruch 3, dadurch gekennzeichnet, daß am Schließteil (25) im Bereich des Verschlußkopfes (27) eine geschwächte Zone vorgesehen ist, die im Falle eines Versuchs der Wiederverwendung reißt.

10. Spritze nach den Ansprüchen 1 und 2, dadurch gekennzeichnet, daß der Kolben (2) quer zur Öffnung (8) des Ringbereichs (7) der Dichtung durch einen axialen Stempel (10) verlängert ist, dessen Durchmesser kleiner ist als derjenige der Öffnung und von einem kopf (11) abgeschlossen wird, der mit dem Stempel eine Schulter (19) ausbildet.

11. Spritze nach den Ansprüchen 1 und 10, dadurch gekennzeichnet, daß oberhalb des Kopfes (11) im Innenraum der Dichtung (4) eine elastische Spange (13) angeordnet ist, die Arme (14) ausbildet, welche im Bereich von Backen (15) zum axialen Stempel (10) zurückspringen und an einem unteren Teilbereich (10b) dieses Stempels anliegen, wobei eine Kehlung (12) von diesem Stempel im wesentlichen im gleichen Abstand zwischen dem Kolben (2) und dem kopf vorgesehen ist.

12. Spritze nach Anspruch 11, dadurch gekennzeichnet, daß der Teilbereich (10b) des Stempels (10) ein im wesentlichen geringfügig konisches Profil aufweist, das dem Profil am Endbereich der Backen (15) entspricht.

13. Spritze nach den Ansprüchen 1, 2 und 11, dadurch gekennzeichnet, daß die Spange (13) in ihrer gespreizten Position verbleibt, bei der ihre Backen (15) einen größeren Abstand als der Durchmesser der Öffnung (8) aufweisen, solange die weiche Membran (7) nicht deformiert wurde.

14. Spritze nach den Ansprüchen 1, 2 und 11, dadurch gekennzeichnet, daß die Deformation der weichen Membran (6) eine Verschiebung der Spange (13), ein Eingreifen der Backen (15) in die Kehlung (12) und die Trennung der Dichtung (4) vom Kolben (2) bewirkt.

15. Spritze nach den Ansprüchen 1, 2 und 11, dadurch gekennzeichnet, daß am Ende des Bewegungshubs des Kolbens die Spange (13) quer durch die Membran in Anschlag an den Rand der Spritze gelangt, was ein Eingreifen der Backen (15) in die Kehlung (12) und ein Trennen der Dichtung (4) vom Kolben (2) bewirkt.

16. Spritze nach Anspruch 10, dadurch gekennzeichnet, daß die Schulter (19) des Kopfes (11) einen größeren Durchmesser als derjenige der Öffnung (8) aufweist, daß der kopf Durchlässe (20) hat, die eine Verbindung des Innenraums (21) der Dichtung (4) mit der öffnung (8) bewirken, und daß weitere Durchlässe (22) im Kolben (2) vorgesehen sind.

17. Spritze nach den Ansprüchen 1 und 16, dadurch gekennzeichnet, daß der kopf (11) an seinem unteren Endbereich mit einem Schneidteil (23) versehen ist, das zur Membran hin gerichtet ist und sich im Inneren der Kammer (21) befindet, solange die weiche Membran (6) nicht deformiert wurde.

18. Spritze nach den Ansprüchen 1 und 17, dadurch gekennzeichnet, daß die Deformation der weichen Membran (6) zu ihrer Perforation am Schneidteil (23) führt und auf diese Weise die Abdichtung der Dichtung (4) aufhebt.

19. Spritze nach den Ansprüchen 1, 2 und 11, dadurch gekennzeichnet, daß die Spange (13) in ihrem unteren Bereich einen Vorsprung (24) aufweist, der eine in der Membran (6) der Dichtung (4) vorgesehene Öffnung durchgreift und die Abdichtung gewährleistet.

## Claims

1. Single injection syringe comprising a cylindrical syringe body at the base of which there can be fixed a sampling and/or injection needle and a piston sliding in the interior of the syringe body, the end of which carries a joint piece sliding in sealed fashion on the internal wall of the syringe, characterised in that the joint piece (4) comprises at least one element (6) which is susceptible to being deformed or displaced starting from its equilibrium position under the action of the pressure which the liquid contained in the syringe exerts during the injection phase and in that the sliding piston (2) is associated with the said joint piece via intermediary members (11, 13, 23, 27) which react to the deformation or to the displacement of the said element and which render the joint piece inoperable for a new filling following the end of the injection.

2. Syringe according to claim 1,
characterised in that the joint piece (4) is a hollow cylindrical member, the lower face of which is formed of a flexible membrane (6) which constitutes the deformable element and which ensures the sealing between the body of the syringe (1) and the chamber (21) at the interior of the joint piece and of which the upper part (7) of annular shape bears against the piston (2).

3. Syringe according to claims 1 and 2,
characterised in that the flexible membrane (1) or the displaceable element carrying an orifice (28) normally closed in a sealed fashion by means of an obturator piece (25, 26) which follows the deformation movement or the displacement movement of the said membrane or of the said element, under the effect of the pressure developed during the injection phase, but which is retained by blocking means (17, 31) in a position where it opens the orifice destroying the sealing between the top and underneath of the joint while the membrane or element returns to its initial position.

4. Syringe according to claim 3,
characterised in that the obturator piece (25) is mounted in the interior of a hollow post (10) carrying an internal shoulder (26) which extends the piston (2) and which itself ends in a sealing head (27) which closes an aperture (28) provided in the membrane (6).

5. Syringe according to claims 3 and 4,
characterised in that the blocking means for the obturator piece (25) are formed by elastic leaves carried by the said piece and folded back against the interior of the hollow post (10), the said leaves being able to hook themselves on the shoulder (26) when the obturator piece has been displaced by the deformation of the membrane (6).

6. Syringe according to claims 3 and 4,
characterised in that the blocking means for the obturator piece (25) are constituted by glue or adhesive located on the piston and on this said piece.

7. Syringe according to claims 1 and 3,
characterised in that the obturator piece (25) is formed by a blister cap (31) held in the interior of the joint piece (4), the concavity of which is directed in the same sense as that of an undeformed membrane (6) of the joint piece and which carries at its centre a sealing head (27) which closes an orifice (28) provided in the membrane.

8. Syringe according to claims 1, 2 and 3,
characterised in that the lateral walls of the joint piece (4) below its annular portion (7) are deformable while the base (6) remains rigid.

9. Syringe according to claim 3,
characterised in that one provides on the obturator piece (25) at the level of the obturation head (47) a zone of weakness susceptible to initiate its rupture in the case of an attempt at re-use.

10. Syringe according to claims 1 and 2,
characterised in that the piston (2) extends across the orifice (8) of the annular portion (7) of the joint piece by means of an axial rod (10) of diameter less than that of the orifice and terminating in a head (11) forming a shoulder (19) with the post.

11. Syringe according to claims 1 and 10,
characterised in that there is mounted above the head (11) at the interior of the joint piece (4) an elastic clip (13) formed with arms (14) which try to return towards the axial post (10) by means of jaws (15) while being supported against lower part (10b) of this post, a throat (12) being provided on the post substantially at an equal distance between the piston (2) and the head.

12. Syringe according to claim 11,
characterised in that the part (10b) of the post (10) has a slightly conical shape corresponding to the shape of the ends of the jaws (15).

13. Syringe according to claims 1, 2 and 11,
characterised in that the clip (13) remains in the position extended for which each of its jaws (15) has spread out to an extent greater than the diameter of the orifice (8) while the flexible membrane (6) has not undergone deformation.

14. Syringe according to claims 1, 2 and 11,
characterised in that the deformation of the flexible membrane (6) ensures the displacement of the clip (13), the engagement of the jaws (15) in the throat (12) and the disintegration of the joint piece (4) and of the piston (2).

15. Syringe according to claims 1, 2 and 11,
characterised in that at the end of the path of movement of the piston, the clip (13) comes to abut across the membrane on the end of the syringe, promoting the engagement of the jaws (15) into the throat (12) and disintegration between the joint piece (4) and the piston (2).

16. Syringe according to claim 10,
characterised in that the shoulder (19) of the head (11) has a diameter superior to that of the orifice (8), the head being traversed by lumens (20) which can communicate the interior chamber (21) of the joint piece (4) with the orifice (8) and in that other lumens (22) are provided in the piston (2).

17. Syringe according to claims 1 and 16,
characterised in that the head (11) is provided at its lower end with a sectioning piece (23) directed in the direction of the membrane and located in the interior of the chamber (21) while the flexible membrane (6) has not been subject to deformation.

18. Syringe according to claims 1 and 17,
characterised in that the deformation of the flexible membrane (6) entrains its perforation on the sectioning member (23) and thus eliminates the sealing function of the joint piece (4).

19. Syringe according to claims 1, 2 and 11,
characterised in that the clip (13) has at its lower part a projection (24) crossing a hole provided in the membrane (6) of the joint piece (4) ensuring sealing.
